# EUROPÄISCHE PATENTANMELDUNG

(11) **EP 2 455 069 A1**
(43) Veröffentlichungstag der Anmeldung: **23.05.2012**
(21) Anmeldenummer: 10190919.0
(22) Anmeldetag: 11.11.2010
(51) Int. Cl.: A61K 9/20, A61K 31/135

(54) **Wirkstofffreie Darreichungsform, Verfahren zu deren Herstellung sowie deren Verwendung**

(71) Anmelder: Laburnum GMBH, 16540 Hohen Neuendorf (DE)
(72) Erfinder: Ronca, Renzo, 16540, Hohen Neuendorf (DE)
(74) Vertreter: Müller & Schubert

(57) **Zusammenfassung**

Die vorliegende Erfindung beschreibt wirkstofffreie Darreichungsformen, Verfahren zu deren Herstellung sowie deren Verwendung zur Herstellung rasch zerfallender wirkstoffhaltiger Arzneiformen.

Die erfindungsgemäßen wirkstofffreien Darreichungsformen, enthalten
mindestens einen Porenbildner, ausgewählt aus Laktosen, Zuckeralkoholen und Cellulosen,
mindestens ein Sprengmittel, ausgewählt aus wasserunlöslichen Stoffen, welche ein hohes Quellvermögen aufweisen, wobei mindestens ein Porenbildner in einer Form vorliegt, welche durch Sprühtrockung erhalten wurde und wobei die Zerfallszeit der wirkstofffreien Darreichungsform in Wasser weniger als 5 Minuten beträgt

Beschrieben wird ferner ein Verfahren zur Herstellung der wirkstofffreien Darreichungsformen und deren Verwendung bei der oralen Applikation von pharmazeutischen Wirkstoffen.

## Beschreibung

Die vorliegende Erfindung betrifft wirkstofffreie Darreichungsformen, Verfahren zu deren Herstellung sowie deren Verwendung zur Herstellung rasch zerfallender wirkstoffhaltiger Arzneiformen.

Die Medikation von Kindern, insbesondere Säuglingen und Kleinstkinder, ist mit großen Problemen behaftet. Zunächst stehen nur wenige geeignete industriell hergestellte Arzneimittel zur Verfügung, welche auf die Physiologie von Kindern abgestimmt sind. Aus diesem Grund werden im zunehmenden Maße entsprechende Medikamenten von Apotheken in Form von Einzelzubereitungen hergestellt, welche individuell auf diese jungen Patienten abgestimmt sind.

In der EP 1 923 056 A1 wird eine pharmazeutische Einzeldosisform beschrieben, welche für die Anwendung bei Kindern besonders geeignet ist. Dabei wird auf einen Träger der zuvor gelöste Wirkstoff in definierten Volumina aufgebracht und somit eine genaue Einzeldosierung je Träger erzielt. Ein Nachteil besteht hierbei darin, dass die Träger, welche dann die definierte Menge des Wirkstoffs enthalten, den Patienten auf oralem Wege verabreicht werden müssen. Hierzu ist es erforderlich, dass die Träger rasch zerfallen, damit diese beispielsweise mittels eines Löffels Säuglingen eingeflößt werden können.

Orale Darreichungsformen, welche Wirkstoffe enthalten, dürfen aber im Allgemeinen nicht zu rasch zerfallen, wenn diese mit Wasser in Berührung kommen. Damit wird vermieden, dass die orale Form bereits im Mund zerfällt und somit nicht mehr zusammen mit dem gleichzeitig getrunkenen Wasser im Ganzen geschluckt werden kann.

Aufgabe der vorliegenden Erfindung ist es daher, eine Darreichungsform zur Verfügung zu stellen, welche einerseits rasch zerfällt, andererseits aber geringe Mengen an wässrigen Lösungen, Suspensionen oder Emulsionen aufnehmen kann ohne dabei zu zerfallen. Die Möglichkeit der Aufnahme wässrigen Lösungen, Suspensionen oder Emulsionen ist notwendig, damit die ansonsten wirkstofffreie Darreichungsform mit dem zu applizierenden Wirkstoff beaufschlagt werden kann.

Die Aufgabe wird durch eine wirkstofffreie Darreichungsform gelöst, welche die Merkmale des Hauptanspruchs aufweist. Vorteilhafte Ausgestaltungen der erfindungsgemäßen wirkstofffreien Darreichungsform sind in den abhängigen Ansprüchen gekennzeichnet.

Gegenstand der vorliegenden Erfindung ist eine wirkstofffreie Darreichungsform, enthaltend mindestens einen Porenbildner, ausgewählt aus Laktosen, Zuckeralkoholen und Cellulosen, mindestens ein Sprengmittel, ausgewählt aus wasserunlöslichen Stoffen, welche ein hohes Quellvermögen aufweisen, wobei mindestens ein Porenbildner in einer Form vorliegt, welche durch Sprühtrockung erhalten wurde und wobei die Zerfallszeit der wirkstofffreien Darreichungsform in Wasser weniger als 5 Minuten beträgt.

Erfindungsgemäß ist bevorzugt, dass diese wirkstofffreie Darreichungsform weitere Porenbildner und Sprengmittel sowie weitere pharmazeutisch annehmbare Füllstoffe und Hilfsstoffe enthält. Die weiteren Porenbildner müssen dabei keine Laktosen, Zuckeralkohole oder Cellulosen sein. Erfindungsgemäß ist ferner bevorzugt, dass der Anteil der Porenbildner am Gesamtgewicht der Darreichungsform 2,5 bis 87,5 Gew.-% beträgt.

Erfindungsgemäß ist auch bevorzugt, dass der Anteil der Sprengmittel am Gesamtgewicht der Darreichungsform 5 bis 35 Gew.-% beträgt.

Erfindungsgemäß besonders bevorzugt ist es, dass die Porenbildner eine Partikelgrößenverteilung besitzen, wobei mindestens 75 Gew.-% der Porenbildner eine Partikelgröße von mehr als 50 µm aufweisen.

Erfindungsgemäß besonders bevorzugt ist eine wirkstofffreie Darreichungsform in Form einer Tablette oder eines Presslings. Dabei ist es besonders bevorzugt, dass mindestens eine der Oberflächen der Tablette oder des Presslings Vertiefungen und/oder Erhöhungen aufweist, welche die Oberfläche vergrößern.

Ein weiterer Gegenstand der vorliegenden Erfindung ist ein Verfahren zur Herstellung einer erfindungsgemäßen wirkstofffreien Darreichungsform, wobei man die Porenbildner, Sprengmittel sowie die weiteren pharmazeutisch annehmbaren Füllstoffe und Hilfsstoffe miteinander mischt und direkt zu einer Tablette oder zu einem Pressling verpresst.

Ein Gegenstand der vorliegenden Erfindung ist ferner die Verwendung einer erfindungsgemäßen wirkstofffreien Darreichungsform zur Herstellung einer mindestens einen Arzneistoff enthaltenden Darreichungsform, wobei man eine Emulsion, eine Suspension oder eine Lösung des Arzneistoffs oder der Arzneistoffe auf die wirkstofffreie Darreichungsform aufbringt und das Lösungsmittel anschließend trocknet.

Erfindungsgemäß besonders bevorzugt ist es dabei, dass man die Emulsion, die Suspension oder die Lösung des Arzneistoffs oder der Arzneistoffe mittels einer Dosiervorrichtung aufbringt und man gegebenenfalls die flüssige Phase der Suspension, die flüchtigen Bestandteile der Emulsion oder das Lösemittel der Lösung mittels Vakuum in die Darreichungsform einsaugt.

Die erfindungsgemäße wirkstofffreie Darreichungsform basiert auf einem Porenbildner, der in der Lage ist, geringe Mengen an Lösemitteln aufzunehmen, ohne dabei zu zerfallen. Es wird angenommen, dass hierbei die Oberflächenstruktur der verwendeten Porenbildner eine entscheidende Rolle spielt. Es wurde gefunden, dass insbesondere sprühgetrocknete Laktosen, Zuckeralkohole und Cellulosen diese Eigenschaften aufweisen. Erfindungsgemäß geeignet sind Laktosen, Zuckeralkohole und Cellulosen und auch deren Derivate und Abkömmlinge. Es hat sich gezeigt, dass eine große Anzahl unterschiedlichster Laktosen, Zuckeralkohole und Cellulosen sowie deren Derivate und Abkömmlinge erfindungsgemäß geeignet sind, die erfindungsgemäßen wirkstofffreien Darreichungsformen zu bilden. Besonders beeignete Porenbildner sind in der Tabelle 1 genannt.

Die erfindungsgemäße wirkstofffreie Darreichungsform ist für die Applikation einer großen Anzahl von Arzneistoffen geeignet. Diese Arzneistoffe gehören einer Vielzahl von Substanzklassen an.

Für Lösungen besonders geeignete Wirkstoffe sind beispielsweise Phenprocoumon, Propranolol, Topiramat, Clobazam und Hydrocortison.

Für Emulsionen besonders geeignete Wirkstoffe sind beispielsweise Carvedilol, Diclofenac-Na und Fludrocortison. Die zu applizierenden Wirkstoffe können als Lösungen, Suspensionen oder Emulsionen auf der Basis von Ethanol (100 %), Ethanol/Wasser, Aceton, Isopropanol, Propylenglykol, MCT, Caprylocaproylmacroglyceriden, Monoethyldiethylenglycolen, Oleoylmacrogolglyceriden und Erdnussöl sowie Mischungen dieser untereinander, nebst Zusätzen von Wasser unter gegebenenfalls Zuhilfenahme von Lösungsvermittlern wie Polyvinylpyrrolidon (PVP), Polysorbat 80, Propylenglycolmonocaprylat und/oder Ammoniak vorliegen.

Die folgenden Beispiele erläutern die Erfindung.

### Beispiel 1

### Allgemeine Arbeitsvorschrift zur Herstellung der erfindungsgemäßen Darreichungsformen

Die Bestandteile werden gemäß den Mengenangeben der Tabelle 1 getrennt eingewogen. Alle Bestandteile, mit Ausnahme der Schmierstoffe, werden im Kubusmischer homogen gemischt. Nach erfolgter Homogenisierung der Pulvermischung werden die Schmierstoffe hinzugegeben und dann nochmals 5 Minuten lang im Kubusmischer gemischt.

Die erhaltene Mischung wird dann direkt verpresst (Exzenter-Tablettenpresse Typ EK 0, Fa. Korsch, Deutschland).

Folgende erfindungsgemäße Darreichungsformen gemäß den Rezepturen 1 bis 10 wurden hergestellt:

**Tabelle 1**

| | **Rezepturen** | | | | | | | | | |
|---|---|---|---|---|---|---|---|---|---|---|
| **Bestandteil** | **1** | **2** | **3** | **4** | **5** | **6** | **7** | **8** | **9** | **10** |
| **Porenbildner** | | | | | | | | | | |
| *alpha Lactose mono-hydrat, agglomeriert* | 37,5 | | | | 5 | | | | 20 | |
| *Cellulose, sprühge-trocknet* | 12,5 | | | | 7,5 | | | 15 | | 2,5 |
| *Mannitol, sprühge-trocknet* | | 60 | 20 | 25 | | 30 | 72,5 | 10 | 50 | 40 |
| *alpha Lactose mono-hydrat, sprühgetrock-net* | 37,5 | | | | 22,5 | | | 45 | | 7,5 |
| *Hypromellose* | | | 3 | | | | | | | |
| *Polyvidonacetat 80 % cum Povidon 19 % cum Na-Lauryl Sulfate cum Silica* | | | | 2,5 | | | | | | |

| **Sprengmittel** | | | | | | | | | | |
|---|---|---|---|---|---|---|---|---|---|---|
| *Vorgekleisterte Mais-stärke* | 10 | 10 | 12,5 | 17 | 15 | 20 | | 20 | 12,5 | 5 |
| *Croscarmellose-Na* | | 10 | 10 | 15 | 15 | 17,5 | 15 | 2,5 | 10 | 15 |
| *Na-Alginat* | | | | 3 | | | | | | |
| *Carmellose-Calcium* | | | | | | | | 2 | | |

| **Füllstoff** | | | | | | | | | | |
|---|---|---|---|---|---|---|---|---|---|---|
| *Isomalt* | | 8 | | | | 10 | | | | 12 |
| *Calcium Hydrogen Phosphate Dihydrate* | | | | | | | | | 5 | |
| *Cellulose, mikrokristal-lin* | | | 17 | 25 | | | | | | 7,5 |
| *Sorbitol* | | | 25 | | | | | | | |
| *ß-Cyclodextrin* | | 10 | 10 | 10 | 30 | 20 | 10 | 3 | | 8 |
| *Siliciumdioxid, hoch-dispers* | 0,125 | | 0,5 | 0,5 | | | | | | 0,5 |

| **Hilfsstoffe (Schmierstoffe)** | | | | | | | | | | |
|---|---|---|---|---|---|---|---|---|---|---|
| *Mg-Stearat* | 0,375 | | | 0,5 | | | | | | |
| *Sacchaorestearat* | 2,0 | 1,0 | 1,5 | 1,5 | 5 | 1 | 1,5 | 1,5 | | 1,5 |
| *Na-stearyl fumarat* | | 1,0 | 0,5 | | | 1,5 | 1 | 1 | 2,5 | 0,5 |
| **Gesamtgewicht (mg)** | 250 - 600 | | | | | | | | | |
| **Bruchfestigkeit (N)** | 1,5-7,5 | | | | | | | | | |
| **Zerfallzeit (sec)** | 60 - 300 | | | | | | | | | |

Die Kontrolle der Bruchfestigkeit wurde mit einem Tablet Tester 6D (Firma Schleuniger, Solothurn, Schweiz) durchgeführt.

Die Zerfallzeit wurde gemäß der Vorschrift des Europäischen Arzneibuchs bestimmt.

### Beispiel 2

### Benetzungstest

Auf die Darreichungsformen der Tabelle 1 wurden jeweils 25 - 250 µl Ethanol/Wasser-Mischungen unterschiedlicher Konzentrationen aufgebracht. Die Darreichungsformen zeigte nach dem Trocken keine sichtbare Veränderung. Insbesondere wurde kein Aufbrechen oder Zerfall der Darreichungsformen beobachtet.

Auf die Darreichungsformen der Tabelle 1 wurden jeweils 50 µl einer Öl-in-Wasser-Emulsion oder einer Öl-in-Ethanol-Emulsion aufgebracht und mittel Vakuum eingesaugt. Die Darreichungsformen zeigte nach dem Trocken keine sichtbare Veränderung. Insbesondere wurde kein Aufbrechen oder Zerfall der Darreichungsformen beobachtet.

### Beispiel 3

### Zubereitung mit dem Wirkstoff Propranolol

Für einen sechs Monate alten Säugling mit 7,1 kg Körpergewicht wird eine Einzeldosis mit 2 mg Propranolol hergestellt. Hierzu wurden 1,25 g Propranolol in 50 ml Ethanol/Wasser (96/4; V/V) unter leichten Erwärmen gelöst.

Gemäß der Rezeptur 1 aus Beispiel 1 wurden Tabletten mit einem Einzelgewicht von 300 mg hergestellt.

Auf die Tabletten gemäß der Rezeptur 1 wurden je 80 µl der Propranolollösung aufgebracht. Die Tabletten wurden im Vakuum (Exsiccator über Silicagel) getrocknet.

Zur Applikation beim Säugling wurde die getrocknete wirkstoffhaltige Tablette in einen Löffel gegeben, in welchem 2 ml Muttermilch/Wasser vorgelegt waren. Die Tablette zerfiel innerhalb von 90 Sekunden. Der Inhalt des Löffels wurde dann dem Säugling eingeflößt.

## Patentansprüche

1. Wirkstofffreie Darreichungsform, enthaltend mindestens einen Porenbildner, ausgewählt aus Laktosen, Zuckeralkoholen und Cellulosen,
mindestens ein Sprengmittel, ausgewählt aus wasserunlöslichen Stoffen, welche ein hohes Quellvermögen aufweisen,
wobei mindestens ein Porenbildner in einer Form vorliegt, welche durch Sprühtrockung erhalten wurde und wobei die Zerfallszeit der wirkstofffreien Darreichungsform in Wasser weniger als 5 Minuten beträgt.

2. Wirkstofffreie Darreichungsform, gemäß Anspruch 1,
**dadurch gekennzeichnet, dass** diese weitere Porenbildner und Sprengmittel sowie weitere pharmazeutisch annehmbare Füllstoffe und Hilfsstoffe enthält.

3. Wirkstofffreie Darreichungsform, gemäß Anspruch 1 oder 2, **dadurch gekennzeichnet, dass** der Anteil der Porenbildner am Gesamtgewicht der Darreichungsform 2,5 bis 87,5 Gew.-% beträgt.

4. Wirkstofffreie Darreichungsform, gemäß mindestens einem der voranstehenden Ansprüche, **dadurch gekennzeichnet, dass** der Anteil der Sprengmittel am Gesamtgewicht der Darreichungsform 5 bis 35 Gew.-% beträgt.

5. Wirkstofffreie Darreichungsform, gemäß mindestens einem der voranstehenden Ansprüche, **dadurch gekennzeichnet, dass** die Porenbildner eine Partikelgrößenverteilung besitzen, wobei mindestens 75 Gew.-% der Porenbildner eine Partikelgröße von mehr als 50 µm aufweisen.

6. Wirkstofffreie Darreichungsform, gemäß mindestens einem der voranstehenden Ansprüche, in Form einer Tablette oder eines Presslings.

7. Wirkstofffreie Darreichungsform, gemäß Anspruch 6,
**dadurch gekennzeichnet, dass** mindestens eine der Oberflächen der Tablette oder des Presslings Vertiefungen und/oder Erhöhungen aufweist, welche die Oberfläche vergrößern.

8. Verfahren zur Herstellung einer wirkstofffreien Darreichungsform, gemäß mindestens einem der voranstehenden Ansprüche, wobei man die Porenbildner, Sprengmittel sowie die weiteren pharmazeutisch annehmbaren Füllstoffe und Hilfsstoffe miteinander mischt und direkt zu einer Tablette oder einem Pressling verpresst.

9. Verwendung einer wirkstofffreien Darreichungsform, gemäß mindestens einem der voranstehenden Ansprüche 1 bis 7, zur Herstellung einer mindestens einen Arzneistoff enthaltenden Darreichungsform, wobei man eine Emulsion, eine Suspension oder eine Lösung des Arzneistoffs oder der Arzneistoffe auf die wirkstofffreie Darreichungsform aufbringt und das Lösungsmittel anschließend trocknet.

10. Verwendung einer wirkstofffreien Darreichungsform, gemäß Anspruch 9, **dadurch gekennzeichnet, dass** man die Emulsion oder die Lösung des Arzneistoffs oder der Arzneistoffe mittels einer Dosiervorrichtung aufbringt und man gegebenenfalls die flüssige Phase der Suspension, die flüchtigen Bestandteile der Emulsion oder das Lösemittel der Lösung mittels Vakuum in die Darreichungsform einsaugt.
